# EUROPEAN PATENT SPECIFICATION

(11) **EP 1 006 968 B1**
(45) Date of publication and mention of the grant of the patent: **02.01.2004**
(21) Application number: 98900919.6
(22) Date of filing: 20.01.1998
(51) Int. Cl.: A61F 11/08

(54) **EARPLUG**
OHRSTÖPSEL
BOUCHON POUR OREILLES

(30) Priority: 21.01.1997 GB 9701188
(43) Date of publication of application: 14.06.2000
(73) Proprietor: CK European Limited, Bexhill-on-Sea, East Sussex TN39 4AJ (GB)
(72) Inventor: CONNOR, Dennis, Frederick, Bexhill-on-Sea East Sussex TN39 3AR (GB)
(74) Representative: Curtis, Philip Anthony
(86) International application number: PCT/GB1998/000165
(87) International publication number: WO 1998/031313

(56) References cited:
- EP-A- 0 580 704
- DE-C- 251 598
- FR-A- 1 215 869
- FR-A- 2 283 662
- FR-A- 2 676 642
- GB-A- 2 173 110
- US-A- 2 246 736

## Description

This invention relates to an earplug, i.e., to a device adapted to fit into an ear usually in order to provide protection against excess excessive noise.

Manufacturing industries, such as the pharmaceutical, food and tobacco industries, are inherently noisy, and it is normal to use earplugs in order to protect workers from the noise. Conventional earplugs comprise a moulded article that is shaped to be inserted in the ear canal; two earplugs can be connected together with a cord or band. The earplugs reduce the pressure level of noise that can reach the ear drum, thereby preventing the wearer from excessive pressure levels of noise. Thus, the earplugs act as a hearing protector.

There is always a risk that an earplug will fall from the wearer's ear into a production line and become mixed with the products being manufactured. In order to deal with this problem it is usual to make earplugs detectable, so that they can be detected by detection equipment located in the production line. It is also usual for earplugs to be coloured blue, so that they can be detected visually.

One of the difficulties with all existing ear plugs is the difficulty to hear human speech when wearing them. This is because the standard deviation (ie the variation in attenuation recorded by a test panel of subjects used in assessing the attenuation performance of the plugs in accordance with ISO4869.1) is high in the range 63 Hz to 1 kHz (the low frequency, vowel sounds, end of speech), causing distortion of the spoken word. This could compromise safety, because a wearer may not be able to hear a spoken warning or instruction.

In US-A-3782379 there is disclosed a method of obtaining room temperature vulcanizable silicone rubber earplugs. This patent specification discloses a method of making earplugs, but it does not deal with the problems of detectability or sound attenuation.

EP-A-0244979 discloses a pair of earplugs connected by a cord. In order to make each earplug detectable, it is provided with a metal ferrule. The structure comprises three flanges extending from a central body. Each flange is in the form of a cup, and the flanges overlap one another.

WO-A-9001914 also discloses a pair of earplugs connected by a cord. In order to make each earplug detectable, it is provided with a metal, magnetic, or X-ray detectable insert. The structure comprises three flanges extending from a central body. Each flange is in the form of a cup, and the flanges overlap one another.

EP-A-0580704 discloses an earplug having an iron powder distributed therein, in order to improve the detectability of the earplug. However, the use of iron in an earplug may cause problems with oxidation.

DE-A-251598 discloses an ear protector comprising an elongated body portion having, in one embodiment, two annular flanges referred to as diaphragms.

According to one aspect of the present invention, we provide an earplug as defined in claim 1.

We have found that the provision of exactly two attenuation flanges, rather than one, three, or more, attenuation flanges gives a low standard deviation at low frequencies of 63 Hz to 1 kHz, and can assist in allowing speech to be understood in noisy work places. In this aspect of the invention, there are no other flanges projecting from the body other than the two attenuation flanges; this is different from the prior art, in which there are usually three attenuation flanges.

Preferably each attenuation flange is substantially cup shaped, and one attenuation flange is larger than another.

The portion of the body adapted to be inserted in the ear preferably has the same diameter as the rest of the body. However, in one embodiment the end portion comprises an enlarged, solid portion. The enlarged solid portion preferably has a diameter less than two thirds of the diameter of either of the attenuation flanges. Typically, the end portion has a diameter about half the diameter of the attenuation flanges. in use, the earplug will be inserted into the ear such that the end portion enters the ear before the flanges.

Preferably each attenuation flange is substantially cup-shaped. One of the attenuation flanges is closer than the other to the end portion to be inserted in the ear. It is preferred that the attenuation flange closer to the ear has a maximum diameter less than the other attenuation flange. It is preferred that the attenuation flange further from the ear has two portions: a first portion which is secured to the body and which curves away from the body in a cup-shaped configuration; and a second portion which is secured to the first portion and which is substantially cylindrical.

The attenuation flanges preferably have a substantially circular cross section. One of the attenuation flanges extends further from the body than the other of the attenuation flanges. Each attenuation flange preferably has a maximum diameter at least 50% greater than the diameter of the body.

Preferably the body is elongate having a substantially constant cross-section along its length. Most preferably the body is substantially cylindrical.

Preferably the attenuation flange further from the ear has a larger radius of curvature than the attenuation flange closer to the ear.

In an embodiment the earplug is provided with means to enable it to be detected by electro-magnetically detectable material, said electro-magnetically detectable material at least partially comprising an alloy of copper and another metal.

It is especially preferred that said alloy is a bronze alloy, such as an alloy of copper with aluminium, manganese or beryllium. In the most preferred embodiment said alloy is an alloy of copper and tin. The alloy may include other metals such as zinc and lead.

The electro-magnetically detectable material may consist solely of a single copper alloy, or it may comprise a mixture of two or more different electro-magnetically detectable materials. In some circumstances it may be desirable for the electro-magnetically detectable material to include iron, but this is not essential.

Advantageously, the electro-magnetically detectable material is in the form of a powder, which is desirably distributed throughout at least a part of the earplug; the distribution of the powder is most preferably homogenous. In practice, the earplug is usually moulded as an integral article, so it is preferred that the electro-magnetically detectable material is distributed, preferably homogeneously distributed, throughout the entire earplug. The homogenous distribution of the electro-magnetically detectable material allows even small fragments of the earplug to be detected with conventional electro-magnetically operating detection equipment.

The earplug according to the invention is adapted to be received in the ear of the wearer, and to this end it is conveniently provided with an appropriately shaped body.

It is preferred that the earplug further comprises a mouldable material. The mouldable material is preferably a polymer, and may be a plastic or rubber. It is especially preferred that the mouldable material is a silicone rubber or a thermoplastic rubber. More preferably the mouldable material is a siloxane polymer. Most preferably, the silicone is a methyl vinyl siloxane polymer. An example of a suitable thermoplastic rubber is available under the trade name Santoprene. These polymers are particularly useful, because they can be produced in any colour, especially blue, and they can be produced with low hardness.

It is preferred that the electro-magnetically detectable material comprises 20 to 60 wt%, more preferably 20 to 50 wt%, and most preferably 40 to 50 wt% of the total weight the materials from which the earplug is moulded.

When the electro-magnetically detectable material is in powder form, then its type and particle size is preferably such as to be detectable in the frequency range 50 kHz to 600 kHz, which is a frequency range that is employed by conventional detection equipment. The particle size of the majority of the powder is preferably below 200 microns, and would usually be above 1 micron. More preferably the particle size of the majority of the powder is in the range 50 to 200 microns and most preferably it is in the range in the range 50 to 100 microns.

The earplug according to the invention can be made by a conventional silicone moulding process. One suitable process is described in US-A-3782379, but the skilled person will appreciate that there are many other suitable processes. The electro-magnetically detectable material can be added to the base materials at the start of the moulding process. There are no special moulding techniques needed to deal with the presence of the electro-magnetically detectable material. When the electro-magnetically detectable material is in powder form, it does not require any special treatment or conditioning in order to reduce the risk of oxidation or migration to the surface.

According to another aspect of the invention there we provide a hearing protector device comprising two of said earplugs, each adapted to be received in a respective one of the ears of a wearer, and connecting means for connecting the earplugs together, and further comprising means to enable the device to be detected by electro-magnetically operating detection equipment, wherein said means comprises an electro-magnetically detectable material, said electro-magnetically detectable material at least partially comprising an alloy of capper and another metal.

The electro-magnetically detectable material is preferably the same as the electro-magnetically detectable material described above.

The connecting means may be any form of cord or band that can be secured to each of the earplugs, preferably at each end of the cord or band. The connecting means is preferably flexible or semi-rigid. Although the connecting means could be completely rigid, a hearing protection device with a rigid connecting means is unlikely to have many practical applications.

A fastener may be provided for fastening the connecting means to each earplug.

The electro-magnetically detectable material may form part of one or more of the earplugs, the fasteners and the connecting means, and is preferably homogeneously distributed therein in powder form.

Reference is made to the accompanying drawing in which:
Fig. 1 is a side view of an embodiment of earplug according to the invention;
Fig. 2 is a top view of the earplug shown in Fig. 1;
Fig. 3 is a view along lines 3-3 of Fig 1;
Fig. 4 is a view along lines 4-4 of Fig. 1; and
Fig. 5 is a bottom view of the earplug shown in Fig. 1.

In the drawings, an earplug generally designated 10 comprises an elongate central body 12 having a solid end portion 14 adapted to be received in the ear of a wearer. An opposite end portion 16 is provided with a recess 18 adapted to receive an earplug connector (not shown). The earplug connector enables two earplugs to be connected together.

Two attenuation flanges 20 and 22 project from the central body 12 intermediate the end portions 14 and 16. Each attenuation flange 20 and 22 is integral with the central body 12.

The attenuation flange 20 is generally cup-shaped and has inner and outer surfaces 24 and 26 respectively. The inner and outer surfaces 24 and 26 extend away from the body 12 along curved lines. The curve of the surfaces 24 and 26 may be, for example, circular or parabolic.

The attenuation flange 22 is also generally cup-shaped and comprises two portions 28 and 30. The portion 28 has inner and outer surfaces 32 and 34 respectively. The inner and outer surfaces 32 and 34 extend away from the body 12 along curved lines. The curve of the surfaces 32 and 34 may be, for example, circular or parabolic. The portion 30 has inner and outer surfaces 36 and 38 respectively. The inner and outer surfaces are substantially cylindrical, and the axes of the cylindrical surfaces 36 and 38 are substantially co-linear with the longitudinal axis of the body 12.

In one preferred embodiment, the length of the body 12 is in the range 20 to 30 mm, prefer ably 26 to 27 mm. The diameter of the body may be 4 to 6 mm, preferably 5 mm. The maximum diameter of the attenuation flange 20 may be 12 to 14 mm, preferably 13 mm, and the maximum diameter of the flange 22 may be 11 to 13 mm, preferably 12 mm.

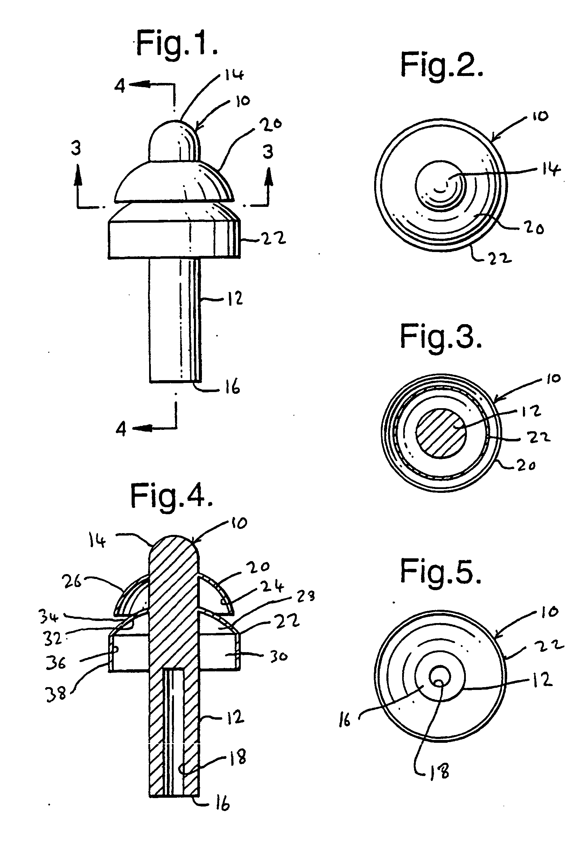

In Fig. 1, the end portion 14 of the body 12 projects beyond the attenuation flange 20. The end portion 14 is preferably 10% to 20%, more preferably 12% to 17%, most preferably 14% to 15%, of the total length of the body. The length of the end portion 14 is typically 3 to 4 mm. The maximum diameter of the attenuation flanges 20 and 22 is preferably less than 40% of the diameter of the body 12.

### Example 1

An earplug having the structure of the earplug 10 in the drawings was made from a mixture of 45 parts by weight spherical particles of tin bronze powder with 55 parts by weight of a silicone. The particles of the bronze powder had a size of 300 mesh.

The earplug was moulded by injection moulding (as an alternative, it could have been moulded by transfer moulding), and was blue in colour - the blue colour was provided by the addition of a blue pigment.

The detectability of the earplug was measured using standard testing procedures which give the detectability in terms of an equivalent size of a non-ferrous sphere. The detectability of the earplug corresponded to a non-ferrous sphere having a diameter of 1.5 mm. This compares very favourably with the silicone plug described in the example of EP-A-0580704, where the detectability corresponded to a metal sphere with a diameter of 2.5 mm.

### Example 2

The attenuation properties of the earplug of Example 1 were testing in accordance with BS EN 24869-1: ISO 4869-1, which specifies a subjective method for measuring the attenuation of hearing protectors at the threshold of hearing.

Thirty four samples of the earplug 10 were supplied. Each tester picked one pair of earplugs at random, and the attenuation was tested once for each subject at a variety of frequencies (in accordance with the procedures specified in BS EN 24869-1:ISO 4869.1). There were a total of sixteen testers. The results were as follows are given in Table 1, and show the attenuation in dB for each tester over a variety of frequencies.

The same test was repeated with a commercially available earplug having three attenuation flanges. The results of the standard deviation obtained with this earplug are given in Table 2.

**TABLE 1**

| Sample | 63 Hz | 125 Hz | 250 Hz | 500 Hz | 1 kHz | 2 kHz | 4 kHz | 5 kHz |
|---|---|---|---|---|---|---|---|---|
| 1 | 20 | 15 | 20 | 22 | 19 | 26 | 26 | 20 |
| 2 | 19 | 20 | 18 | 18 | 21 | 30 | 34 | 38 |
| 3 | 30 | 28 | 26 | 29 | 31 | 39 | 46 | 38 |
| 4 | 16 | 18 | 19 | 19 | 18 | 28 | 31 | 30 |
| 5 | 28 | 24 | 28 | 28 | 26 | 30 | 43 | 30 |
| 6 | 31 | 32 | 28 | 28 | 30 | 39 | 44 | 39 |
| 7 | 18 | 22 | 22 | 18 | 24 | 34 | 38 | 32 |
| 8 | 12 | 15 | 14 | 16 | 24 | 36 | 41 | 42 |
| 9 | 28 | 30 | 21 | 26 | 26 | 32 | 29 | 18 |
| 10 | 17 | 27 | 24 | 21 | 28 | 37 | 27 | 34 |
| 11 | 20 | 20 | 20 | 21 | 19 | 32 | 34 | 26 |
| 12 | 24 | 22 | 21 | 20 | 26 | 35 | 34 | 32 |
| 13 | 27 | 25 | 23 | 24 | 20 | 31 | 33 | 26 |
| 14 | 25 | 22 | 22 | 16 | 22 | 34 | 32 | 29 |
| 15 | 35 | 28 | 24 | 26 | 25 | 41 | 37 | 40 |
| 16 | 14 | 19 | 19 | 20 | 21 | 34 | 25 | 35 |
| Mean | 22.8 | 22.9 | 21.8 | 22 | 23.8 | 33.6 | 34.6 | 31.8 |
| S.D. | 6.7 | 5.1 | 3.7 | 5.1 | 4.0 | 4.2 | 6.5 | 7.0 |

**TABLE 2**

| | 63 Hz | 125 Hz | 250 Hz | 500 Hz | 1 kHz | 2kHz | 4kHz | 5 kHz |
|---|---|---|---|---|---|---|---|---|
| S.D. | 6.4 | 7.9 | 8.2 | 7.6 | 5.6 | 5.5 | 7.5 | 5.9 |

The last two rows in Table 1 show the mean and standard deviation for the results. A comparison of the standard deviations in Tables 1 and 2 shows clearly that the present invention provides a relatively low standard deviation over the frequency range 63 Hz to 1 kHz, which corresponds to human speech.

Modifications can be made to the invention described above. For example, the attenuation flange 22 could have been provided with a continuously curved surface, instead of a curved portion and a cylindrical portion.

## Claims

1. An earplug (10) comprising a body (12) having an end portion (14) adapted to be inserted into an ear, and exactly two attenuation flanges **characterized in that** the attenuation flanges (20, 22) extend in a curved direction away from the body, the arrangement being such that the flange further from said end portion is partly disposed within the attenuation flange closer to said end portion.

2. An earplug according to claim 1, wherein at least part of the surface of at least one attenuation flange (22) is substantially cylindrical.

3. An earplug according to claim 1 or 2, wherein the attenuation flanges have a substantially circular cross section.

4. An earplug according to any one of claims 1 to 3, wherein one (22) of the attenuation flanges extends further from the body than the other of the attenuation flanges (20).

5. An earplug according to claim 4, wherein the attenuation flange that extends further from the body has a radius of curvature greater than a radius of curvature of the other attenuation flange.

6. An earplug according to any one of claims 1 to 5, wherein each attenuation flange has a maximum diameter at least 50% greater than the diameter of the body.

7. An earplug according to any one of claims 1 to 6, wherein the body is elongate and has a substantially constant cross-section along its length.

8. An earplug according to any one of claims 1 to 7, wherein the end of the body that is adapted to be received in an ear projects outwardly from the attenuation flange disposed closest to said end.

9. An earplug according to any preceding claim, wherein the earplug is provided with an electro-magnetically detectable means to enable the earplug to be detected electro-magnetically, said means comprising iron in combination with an alloy of copper with at least one other metal.

10. A hearing protector comprising two earplugs according to any preceding claim, each adapted to be received in a respective one of the ears of the wearer, and connecting means for connecting the earplugs together.

11. A hearing protection device according to claim 10, wherein the connecting means is a flexible cord or band adapted to be secured to each earplug.

## Patentansprüche

1. Ohrstöpsel (10), umfassend einen Körper (12) mit einem Endteil (14) zum Einführen in ein Ohr und genau zwei Dämpfungsflanschen, **dadurch gekennzeichnet, dass** die Dämpfungsflansche (20, 22) sich in einer gekrümmten Richtung vom Körper weg erstrecken, wobei die Anordnung dergestalt ist, dass der von dem genannten Endteil weiter entfernte Flansch teilweise in dem näher an dem genannten Endteil befindlichen Dämpfungsflansch angeordnet ist.

2. Ohrstöpsel nach Anspruch 1, bei dem wenigstens Teil der Oberfläche von wenigstens einem Dämpfungsflansch (22) im Wesentlichen zylindrisch ist.

3. Ohrstöpsel nach Anspruch 1 oder 2, bei dem die Dämpfungsflansche einen im Wesentlichen kreisförmigen Querschnitt haben.

4. Ohrstöpsel nach einem der Ansprüche 1 bis 3, bei dem einer (22) der Dämpfungsflansche sich weiter vom Körper weg erstreckt als der andere der Dämpfungsflansche (20).

5. Ohrstöpsel nach Anspruch 4, bei dem der Dämpfungsflansch, der sich weiter vom Körper weg erstreckt, einen Krümmungsradius hat, der größer ist als ein Krümmungsradius des anderen Dämpfungsflansches.

6. Ohrstöpsel nach einem der Ansprüche 1 bis 5, bei dem jeder Dämpfungsflansch einen maximalen Durchmesser hat, der wenigstens 50 % größer ist als der Durchmesser des Körpers.

7. Ohrstöpsel nach einem der Ansprüche 1 bis 6, bei dem der Körper länglich ist und entlang seiner Länge einen im Wesentlichen konstanten Querschnitt hat.

8. Ohrstöpsel nach einem der Ansprüche 1 bis 7, bei dem das Ende des Körpers, das zum Aufnehmen in einem Ohr ausgeführt ist, von dem am nächsten zu dem genannten Ende angeordneten Dämpfungsflansch nach außen vorspringt.

9. Ohrstöpsel nach einem der vorhergehenden Ansprüche, wobei der Ohrstöpsel mit einem elektromagnetisch entdeckbaren Mittel versehen ist, damit der Ohrstöpsel elektromagnetisch entdeckt werden kann, wobei das genannte Mittel Eisen in einer Kombination mit einer Kupferlegierung mit wenigstens einem weiteren Metall umfasst.

10. Gehörschutz, umfassend zwei Ohrstöpsel nach einem der vorhergehenden Ansprüche, die jeweils zur Aufnahme in einem betreffenden Ohr des Trägers ausgeführt sind, und Verbindungsmittel zum Verbinden der Ohrstöpsel miteinander.

11. Gehörschutzvorrichtung nach Anspruch 10, bei dem das Verbindungsmittel eine biegsame Schnur oder ein biegsames Band zur Befestigung an jedem Ohrstöpsel ist.

## Revendications

1. Un protège-tympan (10) comprenant un corps (12) ayant une partie d'extrémité (14) adaptée pour être introduite dans une oreile, et exactement deux brides d'affaiblissement, **caractérisé en ce que** les brides d'affaiblissement (20, 22) s'étendent en une direction courbe en s'écartant du corps, l'agencement étant tel que la bride la plus distante de ladite partie d'extrémité est partiellement disposée à l'intérieur de la bride d'affaiblissement la plus proche de ladite partie d'extrémité.

2. Un protège-tympan selon la revendication 1, dans lequel une partie au moins de la surface d'au moins une bride d'affaiblissement (22) est sensiblement cylindrique.

3. Un protège-tympan selon la revendication 1 ou 2, dans lequel les brides d'affaiblissement ont une coupe transversale sensiblement circulaire.

4. Un protège-tympan selon l'une quelconque des revendications 1 à 3, dans lequel l'une (22) des brides d'affaiblissement s'étend plus loin du corps que l'autre bride d'affaiblissement (20).

5. Un protège-tympan selon la revendication 4, dans lequel la bride d'affaiblissement qui s'étend le plus loin du corps a un rayon de courbure plus grand qu'un rayon de courbure de l'autre bride d'affaiblissement.

6. Un protège-tympan selon l'une quelconque des revendications 1 à 5, dans lequel chacune des brides d'affaiblissement a un diamètre maximum qui excède de 50% au moins le diamètre du corps.

7. Un protège-tympan selon l'une quelconque des revendications 1 à 6, dans lequel le corps est allongée et a une coupe transversale sensiblement constante sur toute sa longueur.

8. Un protège-tympan selon l'une quelconque des revendications 1 à 7, dans lequel l'extrémité du corps qui est adaptée pour être reçue dans une oreillle fait saillie vers l'extérieur depuis la bride d'affaiblissement disposée le plus près de ladite extrémité.

9. Un protège-tympan selon l'une quelconque des revendications précédentes, dans lequel le protège-tympan est pourvu de moyens détectables électromagnétiquement destinés à rendre possible la détection électromagnétique du protège-tympan, lesdits moyens comprenant du fer, combiné à un cuivre allié avec au moins un autre métal.

10. Un dispositif de protection auditive comprenant deux protège-tympans selon l'une quelconque des revendications précédentes, chacun étant adapté pour être reçu respectivement dans l'une et l'autre des oreilles du sujet porteur, et un moyen de connexion pour connecter les protège-tympans l'un à l'autre.

11. Un dispositif de protection auditive selon la revendication 10, dans lequel le moyen de connexion est un cordon ou ruban flexible adapté pour être fixé à chaque protège-tympan.
